Europäisches Patentamt

European Patent Office

Office européen des brevets

(19)

(11) Publication number: **0 078 422**
**B1**

(12) **EUROPEAN PATENT SPECIFICATION**

(45) Date of publication of patent specification: **17.07.85**

(51) Int. Cl.⁴: **C 07 C 41/42, C 07 C 43/04**

(21) Application number: **82109483.6**

(22) Date of filing: **14.10.82**

(54) Process for the separation of methyl tert-butyl ether from reaction mixtures containing it.

(30) Priority: **20.10.81 IT 2457481**

(43) Date of publication of application:
**11.05.83 Bulletin 83/19**

(45) Publication of the grant of the patent:
**17.07.85 Bulletin 85/29**

(84) Designated Contracting States:
**AT BE DE FR GB NL SE**

(56) References cited:
**EP-A-0 043 478**
**GB-A-2 043 065**
**GB-A-2 049 693**
**US-A-4 144 138**
**US-A-4 198 530**

**Patent Abstracts of Japan, vol. 5, no. 94, 19
June 1981**

(73) Proprietor: **EUTECO IMPIANTI S.p.A.**
**Via Grazioli 11**
**I-20161 Milano (IT)**
(73) Proprietor: **PETROFLEX INDUSTRIA E
COMERCIO S.A.**
**Rua Paraná s/n Campos Eliseos
Duque de Caxias, RJ (BR)**

(72) Inventor: **Trevale, Gaetano**
**Viale Molise 49**
**I-20137 Milano (IT)**
Inventor: **Buzzi, Gian Fausto**
**Via Torino 12**
**I-28041 Arona (Novara) (IT)**

(74) Representative: **Jacobacci, Filippo et al
c/o JACOBACCI-CASETTA & PERANI S.n.c. Via
Alfieri, 17
I-10121 Torino (IT)**

Courier Press, Leamington Spa, England.

## Description

The present invention relates to the separation of methyl tert-butyl ether (MTBE) from the reaction mixture in which it is contained together with methanol and C$_4$-hydrocarbons.

MTBE is a product of great interest, particularly with regard to its use as an anti-knock additive in various types of fuels.

According to the known art MTBE is prepared by reacting isobutylene and methanol in the liquid phase under the influence of acid catalysts, as described, for example, in U.S. Patent Nos. 2,391,084, 2,480,940, 3,121,124, in Belgian Patent No. 612,388 and in the copending Italian application No. 23195-A/81. Usually a hydrocarbon fraction with four carbon atoms (C$_4$-fraction) is used for the purpose, the fraction resulting from the thermal or catalytic pyrolysis of suitable petroleum fractions which contain isobutylene together with linear butenes, butadiene and butanes. The methanol reacts selectively with the isobutylene in the C$_4$-fraction and the reaction proceeds relatively quickly to its thermodynamic equilibrium at moderate temperatures and in the presence of efficient catalysts such as acid sulphonate ion exchange resins.

Given the equilibrium characteristics of the reaction under discussion, the reaction mixture contains considerable quantities of unreacted methanol which must be separated from the MTBE and this separation presents problems as a result of the formation of MTBE-methanol azeotropic mixtures.

It has thus been proposed in the art to wash the MTBE-methanol mixture with water so as to separate an aqueous-alcoholic phase from an ether phase. This method has disadvantages in that the ether layer obtained contains a significant quantity of residual water and also a small proportion of the MTBE remains in the aqueous layer. This involves the use of separation and dehydration treatments which make the process complex and economically onerous.

It has also been proposed to distil the methanol in the mixture with MTBE in the form of an azeotrope with hydrocarbons, particularly C$_4$-hydrocarbons, so as to recover the MTBE as the product at the bottom of the azeotropic distillation column. For this purpose the methanol-MTBE mixtures are enriched with C$_4$-hydrocarbons up to the azeotropic value with methanol and the mixture obtained is distilled, particularly at increased pressures which allow the azeotropic mixtures to be richer in methanol. This method has not resolved the problems of the separation of MTBE-methanol completely. A clear separation is in fact difficult, particularly in the case of mixtures rich in methanol, which, in any case, makes the separation onerous in terms of energy consumption.

Hence the object of the present invention is to provide a process for the separation of methanol from its mixtures with MTBE without, or substantially without, the disadvantages mentioned above.

In accordance with the present invention, methanol is reacted selectively with isobutylene contained in a C$_4$-hydrocarbon fraction, with the use of a methanol/isobutylene molar ratio equal to or greater than 0.7/1, in the liquid phase, under the influence of acidic catalysts and the reaction mixture obtained, containing MTBE and unreacted methanol, is separated in an azeotropic distillation column which is also fed with a lateral flow of a liquid C$_4$-hydrocarbon or a liquid mixture of C$_4$-hydrocarbons such as to maintain a C$_4$-hydrocarbon content in the column which is constantly greater than that in an azeotropic mixture with methanol, and methanol-C$_4$-hydrocarbon which is richer in C$_4$-hydrocarbons than the azeotropic mixture is separated as the product at the head of the column and pure or substantially pure MTBE is separated as the product at the bottom.

Hence, according to the present invention, a methanol-C$_4$-hydrocarbon composition which is richer in C$_4$-hydrocarbons than the azeotropic composition is maintained in the distillation column by feeding a flow of liquid C$_4$-hydrocarbons (for example linear butenes, butanes or their mixtures) laterally to the column at one or more points. It has been found that this device results in a clear separation of the methanol from its mixtures with MTBE with consequent complete recovery of the MTBE in a pure or substantially pure form as the product at the bottom of the column, with the aid of a single distillation operation.

In the preferred embodiment, the lateral flow of liquid C$_4$-hydrocarbons is fed to the first plate from the head of the column as a substitute for the usual reflux. Thus an energy saving compared with the conventional processes for separating methanol and MTBE is also achieved.

The C$_4$-hydrocarbon fractions which are used for the etherification reaction are those which result from the thermal or catalytic pyrolysis of suitable petroleum fractions and which, together with isobutylene, contain variable quantities of linear butenes, butadiene and butanes. The isobutylene content of the said C$_4$-fractions may vary in general from 10 to 55% by volume.

The methanol used in the etherification reaction is conveniently anhydrous, or at least its water content is maintained at values of less than about 0.3% by weight, so as to avoid or at least minimize the formation of byproducts such as tertiary butyl alcohol.

According to the present invention the molar ratio of the methanol to the isobutylene in the feed is maintained at values equal to or greater than 0.7/1, with preferred values in the range 0.8/1 to 1.3/1. With such ratios the oligomerization of the isobutylene is substantially avoided and good values for the conversion of the isobutylene to MTBE are achieved.

The etherification reaction is carried out in the presence of acid catalysts, which, in the preferred embodiment are constituted by solid acid sulphonate ion exchange resins.

The etherification temperatures are generally within a range of from 45 to 100°C. The etherification pressures are those which allow the reaction medium to be maintained in the liquid phase and generally vary from 10 to 40 bar.

In the preferred embodiment, the reagents are supplied and made to flow continuously through the acid sulphonate ion exchange resin which is disposed in the form of a fixed bed, at a space velocity of less than 80 hours$^{-1}$, so that the etherification reaction reaches thermodynamic equilibrium or approximately thermodynamic equilibrium.

The reaction mixture obtained generally contains less than 60% by weight of MTBE and less than 10% by weight of methanol, the remaining percentage being constituted by C$_4$-hydrocarbons.

According to the present invention this mixture is fed to an intermediate point between the head and the foot of a distillation column having 10 to 50 theoretical plates. To one or more points laterally of the column is fed a flow of liquid C$_4$-hydrocarbons, preferably constituted by the same C$_4$-hydrocarbons distilled and freed from methanol. This lateral flow may be supplied to the column in correspondence with the first to the thirtieth plate from the head. The distillation is carried out at a pressure of from 2 to 7 bar, measured at the head of the column.

Within this pressure range the quantity of methanol in the methanol-C$_4$-hydrocarbon azeotrope varies within the range of about 1.5 to 8% by weight. According to the present invention the quantity of C$_4$-hydrocarbons supplied as the lateral flow is metered so that the composition in the distillation column has a higher C$_4$-hydrocarbon content than the azeotropic composition and, in the preferred embodiment, the said lateral flow is supplied, instead of the usual reflux, to the first plate from the head of the column. Under the conditions mentioned, the values of the distillation temperature vary within a range of from 20 to 70°C at the head and from 80 to 150°C at the foot.

The reaction being carried out in the manner indicated, it is possible to separate pure or highly pure (99% or more) MTBE completely at the foot of the column.

The examples which follow are illustrative and non-limiting of the invention. In these examples the parts and percentages are to be understood to be given by weight if not otherwise indicated.

Example 1

To a reactor containing the sulphonate ion exchange resin Amberlyst 15 in the form of a fixed bed of granules of from 16 to 50 mesh, is supplied a liquid flow having the following percentage composition:

| Isobutylene | 36.28 |
| Linear butenes and butanes | 43.90 |
| Methanol | 19.78 |
| Water | 0.04 |

In the reactor the reaction is carried out continuously in the liquid phase at about 55°C, at a pressure of 20 bar and with a space velocity of 5 hours$^{-1}$ and a reaction mixture is discharged having the following percentage composition:

| Isobutylene | 4.85 |
| Linear butenes and butanes | 43.90 |
| Methanol | 2.03 |
| MTBE | 48.82 |
| Tertiary butyl alcohol | 0.15 |
| Dimers and higher oligomers | 0.25 |

This reaction mixture is fed to a distillation column with 21 theoretical plates, operated at a pressure of 5.8 bar measured at the head and with a temperature of about 55°C at the head and about 118°C at the foot.

To the first plate from the head of the column is fed a liquid flow having the following percentage composition:

| Isobutylene | 9.92 |
| Linear butenes and butanes | 89.73 |
| Methanol | 0.35 |

This flow is obtained from the product distilled at the head of the column after condensation and washing with water to remove the methanol.

The weight ratio between the liquid flow fed to the head of the column and the liquid flow coming from the etherification reactor is 0.68/1.

Under the above conditions, the product taken from the bottom of the distillation column consists of MTBE, with a purity of more than 99%, containing traces of tertiary butyl alcohol and oligomers of isobutylene.

MTBE is practically absent from the product at the head of the column.

Example 2

To a reactor containing the sulphonate ion exchange resin Amberlyst 15 in the form of granules of from 16 to 50 mesh, is fed a liquid flow having the following percentage composition:

| Isobutylene | 34.537 |
|---|---|
| Linear butenes and butanes | 43.761 |
| Methanol | 21.643 |
| Water | 0.059 |

In the reactor the reaction is carried out in the liquid phase under conditions similar to those of Example 1 and a reaction mixture is discharged having the following percentage composition:

| Isobutylene | 1.451 |
|---|---|
| Linear butenes and butanes | 43.761 |
| Methanol | 2.849 |
| MTBE | 51.698 |
| Tertiary butyl alcohol | 0.241 |

This reaction mixture is fed to the distillation column of the first example operated at a pressure of 5.8 bar, measured at the head, and with a temperature of about 54°C at the head and about 118°C at the foot.

The vapours which leave the head of the column are condensed and washed with water to remove the methanol. A part of this washed flow is taken off and conveyed to the first plate from the head of the column. The percentage composition of this flow is as follows:

| Isobutylene | 3.19 |
|---|---|
| Linear butenes and butanes | 96.48 |
| Methanol | 0.30 |
| Water | 0.03 |

The weight ratio between the liquid flow fed to the head of the column and the liquid flow coming from the etherification reactor is 0.85/1.

Under the above conditions, the product taken from the bottom of the distillation column consists of MTBE with a purity of more than 99.5% by weight.

The products at the head of the column are practically free from MTBE.

## Claims

1. Process for separation of methyl tert-butyl ether (MTBE), obtained by reacting methanol with isobutylene contained in a $C_4$-hydrocarbon fraction, with the use of a methanol/isobutylene molar ratio equal to or greater than 0.7/1, in the liquid phase, under the influence of acid catalysts characterised in that the reaction mixture containing MTBE and unreacted methanol, is separated in an azeotropic distillation column which is also fed with a lateral flow of a liquid $C_4$-hydrocarbon or a liquid mixture of $C_4$-hydrocarbons such as to maintain a $C_4$-hydrocarbon content in the column which is constantly greater than that in an azeotropic mixture with methanol, and a methanol-$C_4$-hydrocarbon mixture which is richer in $C_4$-hydrocarbons than the azeotropic mixture is separated as the product at the head of the column and pure or substantially pure MTBE is separated as the product at the bottom.

2. Process according to Claim 1, characterised in that the separation is carried out in a column having 10 to 50 theoretical plates under the pressure of from 2 to 7 bar, with a temperature at the foot of 80 to 150°C and at the head of 20 to 70°C, the lateral liquid $C_4$-hydrocarbon flow being fed to at least one point between the first and the thirtieth plate from the head of the column.

3. Process according to Claim 2, characterised in that the said lateral liquid $C_4$-hydrocarbon flow is fed completely to the first plate from the head of the column.

4. Process according to Claim 2, characterised in that the said lateral flow is constituted by the product distilled at the head of the column freed from the methanol content.

## Patentansprüche

1. Verfahren zur Trennung von Methyl-tert-butyläther (MTBE), welcher durch Umsetzung von Methanol mit in einer $C_4$-Kohlenwasserstofffraktion enthaltenem Isobutylen unter Anwendung eines Molverhältnisses Methanol/Isobutylen, das gleich oder größer als 0,7/1 ist, in der flüssigen Phase unter der Einwirkung von sauren Katalysatoren erhalten wurde, dadurch gekennzeichnet, daß man die Reaktionsmischung, die MTBE und nichtreagiertes Methanol enthält, in einer Kolonne zur azeotropen Destillation trennt, welcher auch ein seitlicher Strom aus einem flüssigen $C_4$-Kohlenwasserstoff oder einer flüssigen Mischung von $C_4$-Kohlenwasserstoffen derart zugeführt wird, daß ein $C_4$-Kohlenwasserstoffgehalt in der Kolonne aufrechterhalten wird, welcher konstant größer als jener in einer azeotropen Mischung mit Methanol ist, und daß man eine Methanol-$C_4$-Kohlenwasserstoffmischung, welche an $C_4$-Kohlenwasserstoffen reicher ist als die azeotrope Mischung, als Kopfprodukt der Kolonne und reinen oder im wesentlichen reinen MTBE als Bodenprodukt der Kolonne abtrennt.

2. Verfahren nach Anspruch 1, dadurch gekennzeichnet, daß man die Trennung in einer Kolonne mit 10 bis 50 theoretischen Böden unter dem Druck von 2 bis 7 bar bei einer Temperatur am Fuß der Kolonne von 80° bis 150°C und bei einer Temperatur am Kopf der Kolonne von 20° bis 70°C durchführt, wobei der seitliche Strom aus flüssigen $C_4$-Kohlenwasserstoffen zu mindestens einer Stelle zwischen dem ersten und dem 30. Boden ab dem Kopf der Kolonne zugeführt wird.

3. Verfahren nach Anspruch 2, dadurch gekennzeichnet, daß man den seitlichen Strom aus flüssigen $C_4$-Kohlenwasserstoffen vollständig

dem ersten Boden ab dem Kopf der Kolonne zuführt.

4. Verfahren nach Anspruch 2, dadurch gekennzeichnet, daß der seitliche Strom aus dem Produkt, das am Kopf der Kolonne, befreit vom Methanolgehalt, destilliert, besteht.

**Revendications**

1. Procédé de séparation du méthyl tert-butyl-éther (MTBE), obtenu par réaction du méthanol avec l'isobutylène contenu dans une fraction d'hydrocarbures en $C_4$ en utilisant un rapport molaire méthanol/isobutylène égal ou supérieur à 0,7/1 en phase liquide, sous l'action de catalyseurs acides, caractérisé en ce que le mélange réactionnel contenant le MTBE et du méthanol n'ayant pas réagi, est séparé dans une colonne de distillation azéotropique qui est également alimenté par un courant latéral d'hydrocarbure liquide en $C_4$ ou un mélange liquide d'hydrocarbures en $C_4$ de façon à maintenir dans la colonne une teneur en hydrocarbure en $C_4$ constamment supérieure à celle d'un mélange azéotropique avec le méthanol, et un mélange méthanol-hydrocarbure en $C_4$, plus riche en hydrocarbures en $C_4$ que le mélange azéotropique, est séparé comme produit en tête de colonne, et du MTBE pur ou pratiquement pur est séparé comme produit en bas de colonne.

2. Procédé selon la revendication 1, caractérisé en ce que la séparation est effectuée dans une colonne ayant 10 à 50 plateaux théoriques sous une pression de 2 à 7 bars, à une température au pied de 80 à 150°C et en tête de 20 à 70°C, le courant latéral liquide d'hydrocarbure en $C_4$ étant introduit en au moins un point entre le premier et le trentième plateau à partir de la tête de colonne.

3. Procédé selon la revendication 2, caractérisé en ce que ledit courant latéral liquide d'hydrocarbure en $C_4$ est introduit complètement au niveau du premier plateau en partant de la tête.

4. Procédé selon la revendication 2, caractérisé en ce que ledit courant latéral est constitué par le produit distillé en tête de colonne et débarrassé de sa teneur en méthanol.